# EUROPEAN PATENT SPECIFICATION

(11) **EP 2 175 015 B1**
(45) Date of publication and mention of the grant of the patent: **07.05.2014**
(21) Application number: 09172175.3
(22) Date of filing: 05.10.2009
(51) Int. Cl.: C12M 1/107, B65D 88/34

(54) **Floating cover for tanks for storing liquids**
Schwimmende Abdeckung für Behälter zur Lagerung von Flüssigkeiten
Couvercle flottant pour des réservoirs à liquides

(30) Priority: 07.10.2008 IT CR20080020
(43) Date of publication of application: 14.04.2010
(73) Proprietor: Ecomembrane S.R.L., 26030 Gadesco Pieve Delmona (CR) (IT)
(72) Inventor: Spedini, Luigi, 26100, CREMONA (IT)
(74) Representative: Marcio', Paola

(56) References cited:
- EP-A- 0 350 455
- EP-A- 1 801 037
- WO-A-03/018437
- CH-A5- 662 748
- DE-A1- 3 033 646
- DE-A1- 3 904 326
- DE-A1- 10 115 623
- IT-B- 1 219 578
- US-A- 2 008 686
- US-A- 3 279 606
- DATABASE WPI Week 200851 Thomson Scientific, London, GB; AN 2008-H93935 XP002529110 -& CN 2 928 857 Y ((ZHAO-I) ZHAO X) 1 August 2007 (2007-08-01)

## Description

The invention relates to a cover arranged for floating on the surface of liquid substances contained in open reservoirs and, more in particular, of sewage accumulated in storage tanks, to enable recovery of gases and odorous volatile substances that develop as a result of chemical and physical or biological phenomena.

It is known that during the storage of sewage of residential, industrial or zootechnical origin, volatile substances develop, which tend to be released into the atmosphere, creating problems of emission of odours or gases that are harmful for the environment.

In some cases, said gases have combustible properties and can be used for the production of renewable-type energy. It is hence felt the problem both to prevent the diffusion of disagreeable odours and to recover combustible biological-type gases.

Floating systems used for the purpose of reducing said problems are known, for example by using expanded clay, balls of plastic material or plastic elements with hexagonal shapes, etc.

Such solutions, however, do not enable either the recovery of the gases or the accumulation thereof, but simply reduce the evaporation and the diffusion of odours caused by the wind.

Other known systems relate to the use of floating metal bells that modify their line of floating in relation to the volume of gas contained, running along guides fixed to the wall of the tank.

Nevertheless these systems are expensive, are subject to chemical corrosion, and are not able to follow any possible variations of the level of the liquid contained in the tank, which, for reasons of management and re-use of the sewage, could undergo considerable variations in the course of the year.

Further systems relate to the use of small modules made of plastic floating material connected to one another by flexible pipes arranged for enabling the gases to be transferred between the modules themselves, keeping the pressure constant.

From the Italian patent No. 1 329 922, filed in the name of the present inventor on December 13, 2001, a system is known that can be used for providing covers of tanks containing liquids that employs floating modules for capturing and storing gases and odours that exhale from the liquid itself.

A defect of said system is that the modules are unable to cover the surface of the tank continuously, therefore the volumes of gases that exit from the tank itself are still high.

A further defect is that the positioning or removal of said modules is difficult to obtain, above all as long as the tank is full of liquid.

Another limit is the possibility of recovering rain water only partially.

Yet a further defect is that possible solid substances brought to the surface by the gases that develop from the sewage, above all if it is of animal origin, tend to form a crust that lifts up the modules, preventing withholding of the gases underneath them.

The document No. EP 1 801 037 A discloses a cover for liquid storage tanks, comprising a gas-proof sheet, which is supported by peripheral flexible floating elements and which seals the interior delimited by the cover and the liquid surface so as to allow for collecting gas that may be released from within the liquid beneath the cover. The cover comprises skirts fastened to the top of the floating elements, which are immersed in the liquid beyond the bottom of the floating elements to ensure the sealing of the gas chamber.

The flexible floating elements, however, do not offer any resistance to the radial forces of compression towards the centre of the tank that can develop as a result of the wind or of the gases that are released close to the walls or of floating materials that can slide in between the walls themselves and the sealing skirt. Furthermore, since the sealing skirt is fixed only to the top of the flexible floating elements, it can be lifted by said floating materials, which can drag it upwards or displace it towards the centre of the tank, thus eliminating the effect of impermeable seal.

DE 39 04 326 A1 discloses a gas-proof canopy that is floating on liquid manure and serves to collect nocuous gases. Floating elements are attached to the inner side of the rim of the canopy and the gas-proof sheet extends beyond the floating elements and covers the outer part of the floating elements.

The floating elements, however, do not offer any resistance to the radial forces that can develop as a result of the wind or of the gases collected or of floating materials on liquid manure.

Furthermore, since the sealing sheet is fixed only to the floating elements, when it is not full of gas it lies on the liquid surface and can not take such a form that can be useful both to collect the same gas in the centre from the very beginning, and to enable better drainage of rain water.

The purpose of the present invention is to overcome said defects.

In particular, the purpose of the invention consists in constituting a floating cover arranged for: covering the surface of the tank continuously; covering tanks of any shape; resisting the radial forces and the thrust force towards the centre and upwards caused by the wind, by the gases that develop outside the cover, and by possible solid substances floating on the free surface of the liquid contained in the tank; acting as a gasometer, making available a variable volume for the storage of the gases recovered, guaranteeing the impermeable seal of the cover by means of a skirt that does not rise from the liquid contained in the tank; enabling installation directly on the bottom of the tank if this is empty, or on the outside if this is full, enabling it to be displaced over the sewage in a single lifting operation; enabling the recovery of rain water; enabling to fit possible variations of the level of the liquid contained in the tank, without losing its own functionality; resisting any chemical aggression; functioning as a lid capable of thermal insulation.

The above purposes are achieved with a floating cover for tanks for storing liquids, as claimed in claim 1. Preferred embodiments of the invention are disclosed in the dependent claims.

The invention presents numerous advantages:
- it is made up of elements that can be easily transported, assembled, and are resistant to corrosion;
- it may be adapted to tanks of any shape and dimension;
- it is rigid with respect to radial forces of thrust directed towards the centre of the tank;
- it enables resistance to any thrust directed upwards exerted on the sealing skirt by solid floating materials;
- it enables prevention of accumulation of large quantities of gas underneath the cover when the tank is at the minimum level, thus reducing the problems of safety;
- it enables to work always at a slight pressure so that the sheet will resist the wind and precipitations, even tough the volume of gas contained underneath the cover may vary, thanks to the variation in the floating line;
- it is self-stabilizing and can discharge the gas in excess automatically when the thrust upwards due to the internal pressure causes the exit of the skirt from the sewage;
- it can be positioned and removed even when it is already installed, with the aid of a hoisting crane.

The above and other advantages of the invention will emerge more clearly from the ensuing treatment, in which preferred embodiments are described by way of non-limiting example and with the aid of annexed figures, wherein:
Figure 1 is a cross-sectional view of a floating cover according to the invention positioned on a sewage-storage tank;
Figure 2 illustrates in partially sectioned view from above the floating cover itself;

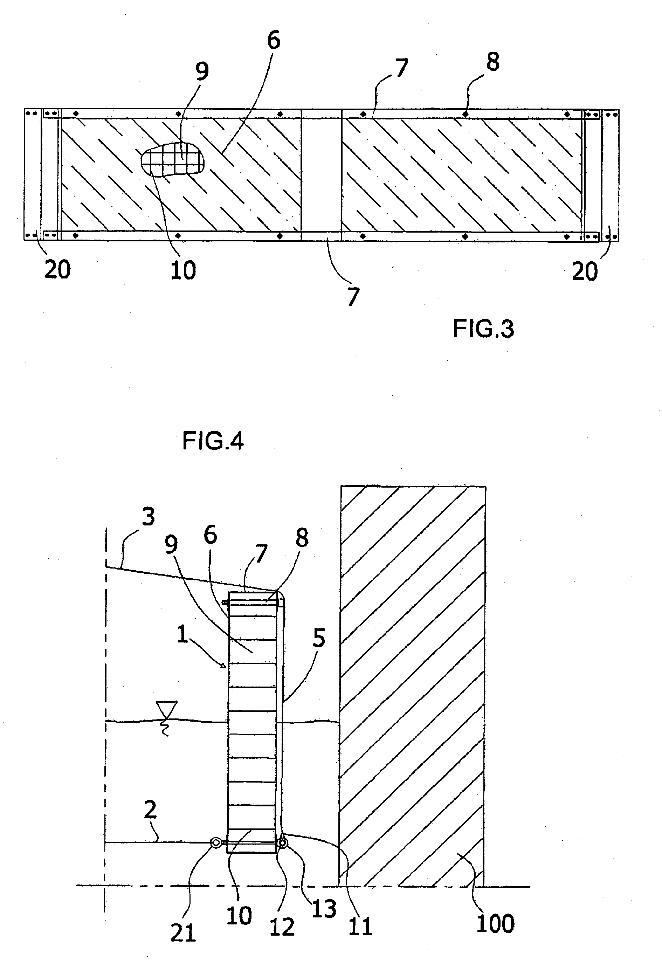

Figure 3 illustrates, in partially sectioned front view, a beam formed by panels constituting one of the floating edge elements;
Figure 4 is a cross-sectional view of a detail of the margin of the sheet that bestrides the floating edge elements;
Figure 5 and 6 illustrate the central floating element in side view and from above, respectively;
Figure 7 illustrates, in side view, a floating cover lifted by a crane.

With reference to the details illustrated in the figures, the floating cover comprises floating edge elements 1, connection cables 2, and a gas-proof sheet 3. The cover also comprises a central floating element 4, fixed to which is a rigid structure that extends vertically for supporting the sheet 3. Said central floating element is arranged for concentrating the gases at the centre and enabling better drainage of rain water.

The floating edge elements 1 are formed by rectilinear beams made up of panels 6 made of polypropylene and by stainless steel sheets 7 bent to form a U, fixed to the panels 6 by means of through bolts 8.

The panels 6 comprise closed cells 9, delimited by diaphragms 10 that join the major faces of the panels themselves according to a substantially square-mesh grid structure.

The dimensions of each panel are, for example, 1 m in length, 0.55 m in height and 0.10 m in thickness. The cells 9 have sides of 0.05 m in length and have hence a substantially parallelepipedal shape with a square base. The thickness of the polypropylene is approximately 3.5 mm.

Such a structural conformation gives the panel a high buoyancy, which is only slightly reduced by the holes made in a few cells to enable the passage of the bolts themselves.

In addition to buoyancy, the panels give the edge elements 1 also a high level of resistance to bending and co-operate structurally with the bent sheets 7 so as to prevent problems of slenderness, enabling concentration of the steel in the areas of greater tensile and compressive stress.

The rectilinear beams constituting the floating edge elements 1 can comprise a plurality of panels 6 set apart from one another by short empty spaces.

In the illustrated variant, the beams are formed by two panels and have a length of approximately 2.4 m.

The rectilinear beams are connected to one another with shaped plates 20 so as to form a polygon of n sides arranged for approximating the shape of the tank 100 to be covered, which in the illustrated example is circular with a diameter of 36 m.

The vertices of the polygon are connected to one another by cables 2 arranged radially, made with steel cables, fixed to the base of the vertices themselves by means of rings 21.

The sheet 3 is made with membranes made of PVC coated polyester-fibre fabric, cut and welded according to the known art, but may also be made of other single-layer or multilayer plastic sheet materials, also of expanded material, according to the requirements, provided that they are sufficiently impermeable to the passage of the gases.

The sheet 3 has a surface development such as to enable it to pass beyond the limit constituted by the top edge of the floating elements 1 and to continue with a skirt 5 external to the polygon delimited by the elements 1 at least till the bottom edge of the elements themselves.

The margin of the skirt 5 is welded on itself so as to constitute a rim 11, which is fixed to the outside of the floating elements 1 by means of rigid tubes 12 that slide, not only into the rim 11, but also into rings 13 arranged along the bottom edge of the rectilinear beams constituting the side of the perimetral polygon.

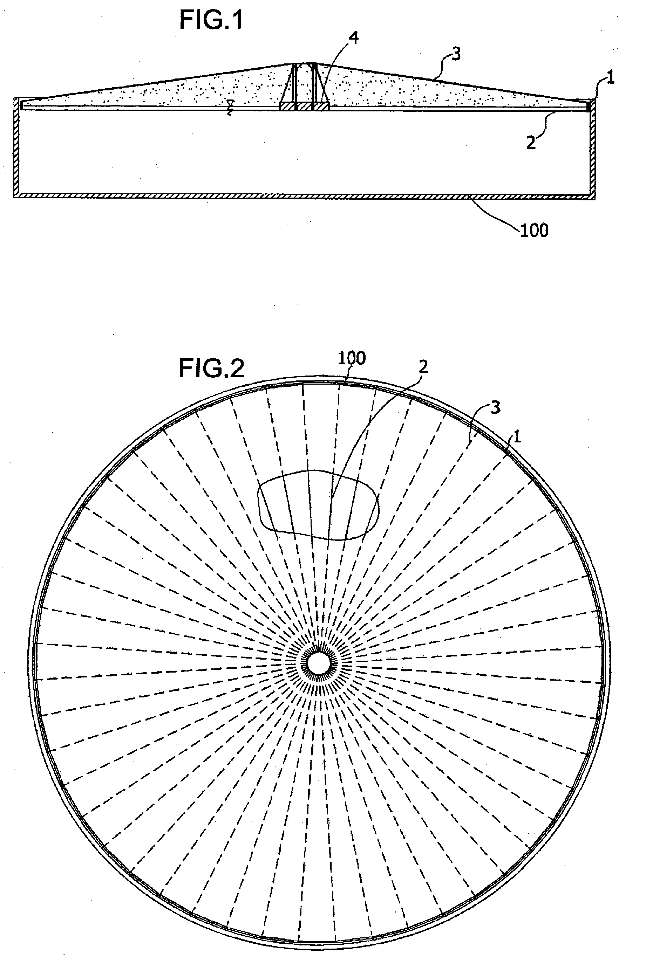

As illustrated in Figures 1 and 2, there is also a central floating structure 4, basically constituted by polypropylene panels 14, similar to the ones used for the edge beams, connected by linear elements 15 and angular elements 16 made of steel so as to be able to contain cubes of closed-cell expanded material 17, for example polystyrene.

Fixed to the floating structure 4 is a rigid-rod structure 18 that terminates at the top with a tubular ring 19 arranged for supporting the sheet 3.

The cables 2 will connect radially the vertices of the perimetral polygon to the bottom of the central floating structure 4.

The presence of the rigid-rod structure 18 requires an adequate conformation of the sheet 3, according to the known art.

If the tank 100 has a central pillar, the sheet 3 will be shaped so as to cap the pillar itself, folding in bellows shape and stretching out thereon according to the floating level of the cover.

The floating cover is completed by accessories of a known type, such as flange joints for connection of pipes for aspiration of the gases. In particular, the position of said gas-outlet points may indifferently be at the centre of the cover or in the proximity of the edge beams according to the presence or otherwise of the central floating structure 4.

On the perimeter of the floating structure there may moreover be applied a gutter arranged for conveying rain water into a sump pit in which a drainage pump is housed. Alternatively, it is possible to position other flange joints on the sheet, which are connected to an underlying network of pipes to enable the rain water to be conveyed into the discharge sump.

At the vertices of the polygon there may be applied horizontal telescopic arms that terminate with a wheel with horizontal axis, arranged for running along the inner wall of the tank to keep the floating cover centred in case the wind tends to displace it.

On the top edge of the floating beams there may be applied a level indicator, for example of the ultrasound type, to measure the degree of emersion of the cover with respect to the level of the liquid contained in the reservoir, which remains visible between the wall and the cover, and for supplying a control signal of possible pumps for the gases extraction.

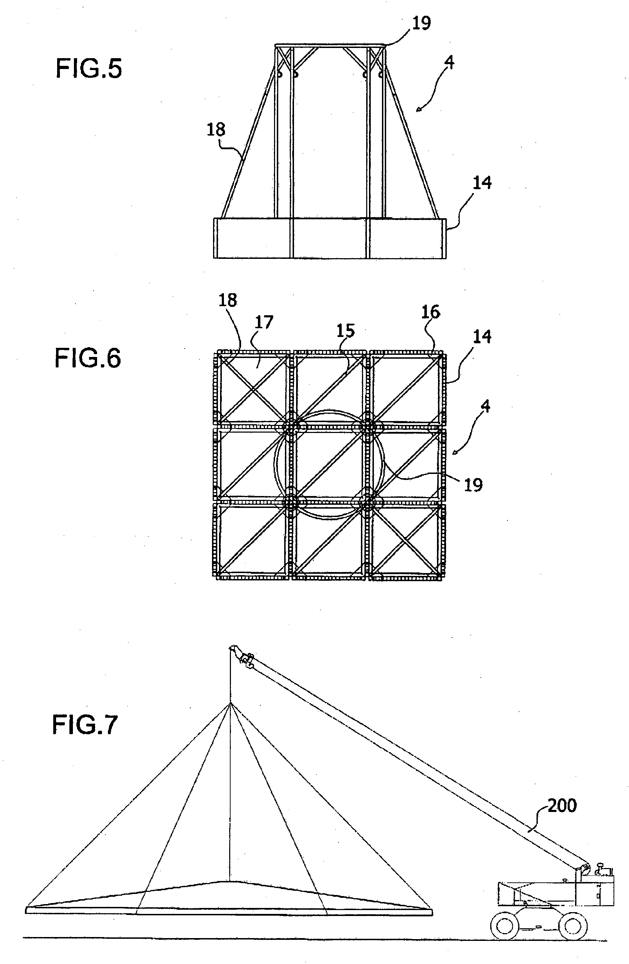

The floating cover can be assembled on the ground and then lifted with a crane 200 and positioned directly on the liquid contained in the tank 100, as illustrated in Figure 7.

To prevent the hoisting cables of the crane from transferring forces of compression to the polygon constituted by the n edge beams, it is possible to use a rigid ring connected to the cables of the crane having the same shape as the edge of the cover, to which the edge of the cover itself may be hung by means of vertical cables.

## Claims

1. A floating cover for tanks (100) for storing liquids, comprising:
- a plurality of floating edge elements (1);
- a gas-proof sheet (3), supported by said floating elements (1), which extends beyond them by means of a skirt (5) and said skirt (5) is fixed to the bottom of said floating elements so as to constitute, together with the liquid contained in the tank (100), a perimetral hydraulic guard, arranged for keeping gases and volatile substances underneath the sheet (3);
said floating cover being **characterized in that** it comprises a central floating structure (4) arranged for supporting the sheet (3), and cables (2) arranged radially, which connect said floating elements (1) to said central floating structure (4).

2. A floating cover according to claim 1, **characterized in that** said floating edge elements (1) comprise hollow plastic panels (6).

3. A floating cover according to claim 2, **characterized in that** said panels (6) are joined together by metal sheets (7) that are bent into a "U" shape so as to produce composite rectilinear beams.

4. A floating cover according to claim 2, **characterized in that** the hollow structure of said panels (6) comprises closed cells (9), delimited by diaphragms (10) arranged to form a regular grid.

5. A floating cover according to claim 3, **characterized in that** the ends of said beams are rigidly connected to one another so as to form a n-sided polygon that approximates the shape of the surface of the tank (100) to be covered.

6. A floating cover according to claim 5, **characterized in that** said cables (2) connect the vertices of said n-sided polygon.

7. A floating cover according to claim 6, **characterised in that** said cables (2) are positioned in a horizontal plane that passes through the bottom of said beams.

8. A floating cover according to claim 1, **characterized in that** the skirt (5) of the sheet (3) is stably fastened to the bottom of the floating elements (1) by means of rigid tubes (12) that slide simultaneously into a rim (11) provided along the margin of the skirt (5) and rings (13) arranged along the bottom edge of said floating elements (1).

9. A floating cover according to claim 5, **characterized in that** said cables (2) radially connect the vertices of said n-sided polygon to said central floating structure (4).

## Patentansprüche

1. Eine Schwimmdecke für Tanks (100) zur Lagerung von Flüssigkeiten, umfassend:
- eine Vielzahl von schwimmenden Randelementen (1);
- eine gasdichte Abdeckfolie (3), gestützt von den besagten Schwimmelementen (1), die sich über diese hinaus mittels einer Einfassung (5) erstreckt, und die besagte Einfassung (5) ist am Boden der besagten Schwimmelemente so befestigt, dass sie, zusammen mit den im Tank (100) enthaltenen Flüssigkeiten, einen umlaufenden hydraulischen Schutz bildet, der so eingerichtet ist, dass er Gase und flüchtige Stoffe unter der Abdeckfolie (3) hält;
wobei die besagte Schwimmdecke **dadurch gekennzeichnet ist, dass** sie einen zentralen Schwimmkörper (4) umfasst, der so eingerichtet ist, dass er die Abdeckfolie (3) und die radial angeordneten Kabel (2), die die besagten Schwimmelemente (1) mit dem besagten zentralen Schwimmkörper (4) verbinden, stützt.

2. Eine Schwimmdecke gemäß Anspruch 1, **gekennzeichnet dadurch, dass** die besagten schwimmenden Randelemente (1) hohle Kunststoffpaneele (6) umfassen.

3. Eine Schwimmdecke gemäß Anspruch 2, **gekennzeichnet dadurch, dass** die besagten Paneele (6) durch Metallbleche (7) miteinander verbunden sind, die so in "U" Form gebogen sind, dass sie geradlinige Verbundbalken bilden.

4. Eine Schwimmdecke gemäß Anspruch 2, **gekennzeichnet dadurch, dass** die hohle Struktur der besagten Paneele (6) geschlossene Zellen (9) umfasst, die von Membranen (10) begrenzt werden, die so eingerichtet sind, dass sie ein regelmäßiges Raster bilden.

5. Eine Schwimmdecke gemäß Anspruch 3, **gekennzeichnet dadurch, dass** die Enden der besagten Balken starr miteinander verbunden sind, so dass sie ein *n*-seitiges Polygon bilden, das annähernd die Form der Oberfläche des zu bedeckenden Tanks (100) besitzt.

6. Eine Schwimmdecke gemäß Anspruch 5, **gekennzeichnet dadurch, dass** die besagten Kabel (2) die Spitzen des besagten n-seitigen Polygons miteinander verbinden.

7. Eine Schwimmdecke gemäß Anspruch 6, **gekennzeichnet dadurch, dass** die besagten Kabel (2) in einer horizontalen Ebene liegen, die durch den Boden der besagten Balken läuft.

8. Eine Schwimmdecke gemäß Anspruch 1, **gekennzeichnet dadurch, dass** die Einfassung (5) der Abdeckfolie (3) am Boden der Schwimmelemente (1) mittels fester Rohre (12) dauerhaft befestigt ist, die simultan in einen am Rand der Einfassung (5) befindlichen Deckelring (11) und in Ringe (13) gleiten, die an der Unterkante der besagten Schwimmelemente (1) angeordnet sind.

9. Eine Schwimmdecke gemäß Anspruch 5, **gekennzeichnet dadurch, dass** die besagten Kabel (2) die Spitzen des besagten n-seitigen Polygons mit dem besagten Schwimmkörper (4) radial verbinden.

## Revendications

1. Couvercle flottant pour des réservoirs (100) à liquides, comprenant :
- une multitude d'éléments à bord flottant (1) ;
- une bâche étanche au gaz (3), soutenue par lesdits éléments flottants (1), qui dépasse de ces derniers à l'aide d'une jupe (5) et ladite jupe (5) est fixée au fond desdits éléments flottants de manière à constituer, conjointement au liquide contenu dans le réservoir (100), une barrière hydraulique périmétrique prévue pour maintenir les gaz et les substances volatiles sous la bâche (3) ;
ledit couvercle flottant étant **caractérisé par le fait qu'**il comprend une structure flottante centrale (4) prévue pour soutenir la bâche (3), et des câbles (2) disposés de manière radiale, qui relient lesdits éléments flottants (1) à ladite structure flottante centrale (4).

2. Un couvercle flottant selon la revendication 1, **caractérisé par le fait que** lesdits éléments à bord flottant (1) comprennent des panneaux en plastique creux (6).

3. Un couvercle flottant selon la revendication 2, **caractérisé par le fait que** lesdits panneaux (6) sont reliés ensembles par des tôles métalliques (7) qui sont courbées en forme de « U » de manière à produire des faisceaux rectilignes composites.

4. Un couvercle flottant selon la revendication 2, **caractérisé par le fait que** la structure creuse desdits panneaux (6) comprend des cellules fermées (9), délimitées par des membranes (10) disposées de manière à former une grille régulière.

5. Un couvercle flottant selon la revendication 3, **caractérisé par le fait que** les extrémités desdits faisceaux sont reliées de façon rigide l'une à l'autre de manière à former un polygone à *n* côtés ayant approximativement la forme de la surface du réservoir (100) à couvrir.

6. Un couvercle flottant selon la revendication 5, **caractérisé par le fait que** lesdits câbles (2) raccordent les sommets dudit polygone à *n* côtés.

7. Un couvercle flottant selon la revendication 6, **caractérisé par le fait que** lesdits câbles (2) sont placés dans un plan horizontal qui passe à travers le fond desdits faisceaux.

8. Un couvercle flottant selon la revendication 1, **caractérisé par le fait que** la jupe (5) de la bâche (3) est fixée de manière stable au fond des éléments flottants (1) à l'aide de tubes rigides (12) qui coulissent simultanément dans une bordure (11) prévue le long de la marge de la jupe (5) et d'anneaux (13) disposés le long du bord inférieur desdits éléments flottants (1).

9. Un couvercle flottant selon la revendication 5, **caractérisé par le fait que** lesdits câbles (2) relient de manière radiale les sommets dudit polygone à *n* côtés à ladite structure flottante centrale (4).
